# EUROPÄISCHE PATENTSCHRIFT

(11) **EP 1 605 952 B1**
(45) Veröffentlichungstag und Bekanntmachung des Hinweises auf die Patenterteilung: **02.05.2007**
(21) Anmeldenummer: 03816433.1
(22) Anmeldetag: 20.11.2003
(51) Int. Cl.: A61K 31/7072, A61K 31/664, A61P 35/00

(54) **VERWENDUNG VON 5-SUBSTITUIERTEN NUKLEOSIDEN ZUR VERSTÄRKUNG DER APOPTOTISCHEN WIRKUNG VON ZYTOSTATIKA**
USE OF 5-SUBSTITUTED NUCLEOSIDES FOR REINFORCING THE APOPTOTIC EFFECT OF CYTOSTATIC DRUGS
UTILISATION DE NUCLEOSIDES SUBSTITUÉS EN 5 POUR RENFORCER L'EFFET APOPTOTIQUE DE CYTOSTATIQUES

(30) Priorität: 24.03.2003 DE 10313035
(43) Veröffentlichungstag der Anmeldung: 21.12.2005
(73) Patentinhaber: Resprotect GmbH, 01307 Dresden (DE)
(72) Erfinder: FAHRIG, Rudolf, 30657 Hannover (DE); HEINRICH, Jörg-Christian, 01309 Dresden (DE); KRUPITZA, Georg, A-1160 Wien (AT)
(74) Vertreter: Pfenning, Meinig & Partner GbR
(86) Internationale Anmeldenummer: PCT/EP2003/013008
(87) Internationale Veröffentlichungsnummer: WO 2004/084917

(56) Entgegenhaltungen:
- WO-A-01/07088
- WO-A-96/23506
- CLERCQ DE E: "POTENTIAL OF BROMOVINYLDEOXYURIDINE IN ANTICANCER CHEMOTHERAPY" ANTICANCER RESEARCH, HELENIC ANTICANCER INSTITUTE, ATHENS,, GR, Bd. 6, Nr. 4, Juli 1986 (1986-07), Seiten 549-557, XP001070144 ISSN: 0250-7005
- FAHRIG, RUDOLF ET AL: "Prevention of adriamycin-induced mdr1 gene amplification and expression i mouse leukemia cells by simultaneous treatment with the anti-recombinogen bromovinyldeoxyuridine" ANTI-CANCER DRUG DESIGN (2001), VOLUME DATE 2000, 15(5), 307-312 , XP008030116
- IIGO M ET AL: "EFFECT OF (E)-5-(2-BROMOVINYL)-2'-DEOXYURIDINE ON LIFE-SPAN AND 5-FLUOROURACIL METABOLISM IN MICE WITH HEPATIC METASTASES" EUROPEAN JOURNAL OF CANCER, PERGAMON PRESS, OXFORD, GB, Bd. 26, Nr. 10, 1990, Seiten 1089-1092, XP008030091 ISSN: 0959-8049
- DEGREVE, B. ET AL: "Selection of HSV-1 TK gene-transfected murine mammary carcinoma cells resistant to (E)-5-(2-bromovinyl)-2'-deoxyuridine ( BVDU and ganciclovir (GCV)" GENE THERAPY (2000), 7(18), 1543-1552 , XP001190852
- BALZARINI J ET AL: "INCREASED SENSITIVITY OF THYMIDINE KINASE-DEFICIENT (TK-) TUMOR CELL LINES TO THE CELL GROWTH INHIBITORY EFFECTS OF (E)-5-(2-BROMOVINYL)-2'-DEOXYURIDINE (BVDU) AND RELATED COMPOUNDS" ANTICANCER RESEARCH, HELENIC ANTICANCER INSTITUTE, ATHENS,, GR, Bd. 6, Nr. 5, 1986, Seiten 1077-1084, XP008030090 ISSN: 0250-7005
- PANCHEVA S N: "METHOTREXATE POTENTIATES ANTI-HERPES SIMPLEX VIRUS TYPE 1 ACTIVITY OF E-5-(2-BROMOVINYL)-2'-DEOXYURIDINE" ACTA VIROLOGICA, ACADEMIA PRAGUE, PRAGUE, CS, Bd. 39, Nr. 2, 1995, Seiten 117-119, XP008010809 ISSN: 0001-723X

## Beschreibung

Die Erfindung betrifft die Verwendung von BVDU zur Herstellung eines Arzneimittels zur Verstärkung der apoptotischen Wirkung von Zytostatika nach der Chemotherapie, bei der mindestens ein Zytostatikum mit BVDU und/oder dessen Salzen eingesetzt worden ist.

Krebserkrankungen sind beim Menschen eine der häufigsten Todesursachen und die Chemotherapie ist die häufigste Behandlungsmethode. Die unzureichenden Heilungschancen durch eine Chemotherapie beruhen auf dem Auftreten von Resistenzen. Diese Resistenzen haben ihre Ursache darin, dass Zytostatika die Expression von Genen beeinflussen und genotoxisch wirken, also Mutationen, Genamplifikationen und Rekombinationen induzieren und somit das Genom instabilisieren. Auf diese Weise induziert oder selektiert eine Chemotherapie resistente Krebszellen. Von solchen durch Zytostatika induzierten Wirkungen sind oft Onkogene, wie z.B. *Ras, Bcl2, Bcr-abl, Myc, ErbB2*, und andere betroffen. Zur Chemoresistenz trägt auch die fehlregulierte Expression von Genen im Zusammenhang mit DNA-Reparatur und Rekombination bei (z.B. p53 Gen, *BRCA1*/*2, UBE2N, APEX* und *Rad51*), weiterhin Enzyme, die Zytostatika metabolisieren und bioaktivieren (z.B. DHFR, DT-diaphorase (DT-D), oder Proteine, die Zytostatika transportieren (z.B. MDR1).

Die meisten Zytostatika eliminieren Tumorzellen dadurch, dass sie Apoptose induzieren. Apoptose ist eine Form des programmierten Zelltods, die erstmals in Kerr, J.F. et al., Br J Cancer, 26(4) (1972); 239-257. beschrieben wurde. Die Apoptose ist im Gegensatz zur Nekrose eine physiologische Form des Zelltods. Anhand von Unterschieden zwischen Nekrose und Apoptose lassen sich diese beiden Formen des Zelltods differenzieren. Apoptose hat definierte morphologische und biochemische Charakteristika, die als Ereignisse einer geordneten Kaskade nacheinander ablaufen. Der kontinuierliche Prozess lässt sich in Phasen einteilen. Morphologische Kennzeichen der Apoptose sind Kernchromatinkondensation (Karyopyknosis), Schrumpfung des Zytoplasma, Formierung apoptotischer Bläschen und schließlich apoptotischer Körper. Tumorzellen können dies durch eine Überaktivierung von Überlebens-Mechanismen verhindern. Mechanismen der Chemoresistenz umfassen also auch anti-apoptotisch wirkende Gene wie z.B. STAT3, den aktivierten "signal transducer and activator of transcription 3" oder JUN-D.

1995 wurden bis dahin unbekannte Wirkungen bestimmter Hormone und 5-substituierter Nukleoside entdeckt. Diese unterdrücken die 2-Amino-6-mercaptopurin (AMP)-induzierte SV40-Amplifikation in Zellen des chinesischen Hamsters (Fahrig, R. et al., Mutat Res., 356 (2), 1996, 217-224) und die Triethylenmelamin-induzierte Rekombination in Hefen (Fahrig, R., Mutat Res, 372 (1), 1996, 133-139). In der EP 0 806 956 B1 wird die Behandlung von Leukämiezellen der Maus mit 5-substituierten Nukleosiden beschrieben, wobei die Doxorubicin (Adriamycin)-induzierte Mdrl-Genamplifikation und Chemoresistenz gehemmt wurde.

In den bislang durchgeführten in vitro-Untersuchungen wurden 5-substituierte Nukleoside (d.h. Basenanaloga) immer zusammen mit einem oder mehreren Zytostatika appliziert.

Aus der WO 01/070 88A ist ferner die Verwendung von BVDU zusammen mit Fluoropyrimidinen oder Tomudex zur Behandlung von resistenten Zellen bekannt.

Dokumente:
WO 96/23506 A (FAHRIG RUDOLF; FRAUNHOFER GES FORSCHUNG (DE); STEINKAMP ZUCHT ANGE) 8. August 1996
CLERCQ DE E: "POTENTIAL OF BROMOVINYLDEOXYURIDINE IN ANTICANCER CHEMOTHERAPY" ANTICANCER RESEARCH, HELENIC ANTICANCER INSTITUTE, ATHENS" GR, Bd. 6, Nr. 4, Juli 1986 (1986-07), Seiten 549-557, XP001070144
und
FAHRIG, RUDOLF ET AL: "Prevention of adriamycin-induced mdr1 gene amplification and expression i mouse leukemia cells by simultaneous treatment with the anti-recombinogen bromovinyldeoxyuridine" ANTI-CANCER DRUG DESIGN (2001), VOLUME DATE 2000, 15(5), 307-312 , XP008030116
beschreiben die gleichzeitige Verabreichung von BVDU zusammen mit anderen antineoplastischen Verbindungen zur Vorbeugung der Resistenzbildung gegenüber Zytostatikabehandlung: sie zeigen Synergismus von BVDU mit Zytostatika, wenn gleichzeitig verabreicht.

Ausgehend von dem hier beschriebenen Stand der Technik war es Aufgabe der vorliegenden Erfindung, die durch Resistenzbildung bedingte Abnahme der apoptotischen Wirkung zu verhindern oder zumindest zu verzögern und hierbei eine verbesserung gegenüber den aus dem Stand der Technik bekannten Therapieformen bereitzustellen.

Diese Aufgabe wird durch die in Anspruch 1 beschriebene Verwendung gelöst. Die weiteren abhängigen Ansprüche zeigen vorteilhafte Weiterbildungen auf.

Erfindungsgemäß wird die Verwendung von BVDU zur Herstellung eines Arzneimittels zur Verstärkung der apoptotischen Wirkung von Zytostatika nach der Chemotherapie bereitgestellt, wo in der Chemotherapie mindestens ein Zytostatikum in Verbindung mit BVDU eingesetzt worden ist.

Erfindungsgemäß wurde bei der Chemotherapie bereits mindestens ein Zytostatikum in Verbindung mit einem BVDU enthaltenden Arzneimittel eingesetzt.

BVDU [(E)-5-(2-Bromovinyl)-2'-Deoxyuridin], kann auch in Form ihrer, Salze verwendet werden. Ein Beispiel für ein erfindungsgemäßes Prodrug von BVDU ist in der allgemeinen Formel I dargestellt:

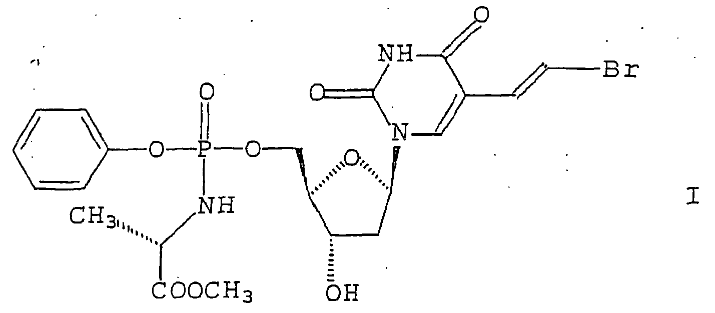

Vorzugsweise wird BVDU in einer Dosierung eingesetzt, die zu einer Blutkonzentration zwischen 0,02 und 50 µg/ml führt.

Es konnte überraschend gezeigt werden, dass nach durchgeführter Chemotherapie, wenn die Zellen allein mit BVDU weiter wachsen, deren Wachstum noch mehr gehemmt wird, als wenn die Chemotherapie mit Zytostatika fortgeführt worden wäre. Völlig unerwartet verstärkte sich der Effekt der 5-substituierten Nukleoside (BVDU) anstatt nachzulassen.

Diese Wirkung wurde mittels eines Screeningsystems festgestellt. Dieses Screening-Verfahren beruht auf der Behandlung von Tumorzellen während eines Chemotherapiezyklus über einen Zeitraum von vorzugsweise 8 bis 30 Tagen mit steigenden Dosen eines Zytostatikums und einer gleich bleibenden Dosis des Überexpressionshemmers (BVDU). Nach dieser Kombination wird das Zytostatikum abgesetzt und die Behandlung allein mit dem BVDU fortgesetzt. Diese Erholungsphase (auch Recovery-Phase genannt) dauert vorzugsweise zwischen 3 und 10 Tagen. Derartige Chemotherapiezyklen können bis zu 6 mal nacheinander durchgeführt werden.

Somit ergab sich eine für den Fachmann überraschende Konstellation der Behandlungsformen:
- 5-substituierte Nukleoside (BVDU), für sich allein gegeben, zeigen keine Wirkung.
- 5-substituierte Nukleoside (BVDU), zusammen mit einem Zytostatikum gegeben, zeigen eine Wirkung.
- 5-substituierte Nukleoside (BVDU), für sich allein gegeben, nachdem sie zuvor zusammen mit einem Zytostatikum gegeben worden waren (Erholungsphase oder Recovery Phase), zeigen eine verstärkte Wirkung.

Die Wirkung, d.h. die Hemmung von Chemoresistenz und Erhöhung der Chemosensitivität, lässt sich als atoxische Aufrechterhaltung Zytostatika induzierter Apoptose durch Beeinflussung der Genexpression bestimmter Gene beschreiben. Dies geschieht durch
1. Blockade von "survival pathways" in der Erholungsphase (recovery).
2. Blockade von DNA-Reparatur assoziierter Enzymen.
3. Induktion von DT-Diaphorase Aktivität.
4. Reduzierte Expression ATP-generierter Enzyme in der Erholungsphase.

Zu 1) Basenanaloga wie BVDU blockieren "survival pathways" vorrangig in der Erholungsphase der Kobehandlung nach Absetzen der Zytostatika und erzwingen dadurch den Ablauf der Apoptose.

Mittels HOPI-Doppelfärbung von AH13r Tumorzellen der Ratte konnte nachgewiesen werden, dass Zytostatika wie Doxorubicin (DOX), Mitoxantron (MXA) oder Mitomycin C (MMC) Apoptose auslösen. Kobehandlung mit dem Basenanalogon (E)-5-(2-bromovinyl)-2'-deoxyuridine (BVDU) fördert die Apoptose durch Blockade anti-apoptotischer "survival pathways", die STAT3 und JUN-D umfassen.

Dieser Effekt tritt erst in der Erholungsphase (recovery phase) der Zellen auf, wie in Referenz-Beispiel 2 zu sehen ist.

Konstitutiv aktiviertes Stat3 wirkt onkogen und trägt zur Entwicklung verschiedener menschlicher Krebserkrankungen bei. Dies geschieht durch Hemmung von Apoptose. Auf diese Weise erleichtert STAT3 das Überleben von Tumorzellen und macht Zellen resistent gegenüber einer Chemotherapie. Dementsprechend induziert die Hemmung des "Stat3-signaling" Apoptose und erhöht die Sensitivität gegenüber Zytostatika.

Jun-D, ein Mitglied der Jun-Genfamilie, ist ein essentieller Bestandteil des "activating protein-1" (AP-1) Transkription-Faktor-Komplexes mit allgegenwärtiger Expressivität. Jun-D^{(-/-)} primäre Fibroblasten zeigen vorzeitige Alterung und erhöhte Apoptose nach UV-Bestrahlung oder TNFα-Behandlung. Dieses Resultat lässt vermuten, dass JUN-D den "NFkappaB survival pathway" aktiviert. Darüber hinaus macht p202, welches direkt durch JUN-D reguliert wird, Fibrobiasten widerstandsfähiger gegenüber Apoptose.

Kobehandlung durch BVDU reduzierte die Expression beider JUN-D-Isoformen um etwa ein Viertel. Demgegenüber war STAT3 in der Erholungsphase (recovery phase) um etwa zwei Drittel reguliert (Referenz-Beispiel 2).

Besonders eindrucksvoll ist der Effekt in der Erholungsphase nach Kobehandlung mit Mitomycin C. Hier reduziert das Basenanalogon die Überexpression des Onkogens JUN-D auf das Kontrollniveau (Referenz-Beispiel 2).

Zu 2) Basenanaloga wie BVDU blockieren DDX1. DDX1 ist coamplifiziert mit MYCN und überexprimiert in Neuroblastoma (NB) und Retinoblastoma Zelllinien und Tumoren. NB Patienten mit Amplifikation von sowohl DDX1 als auch MYCN haben eine schlechtere Prognose als Patienten mit nur MYCN Genamplifikation. DDX1 hat deshalb onkogenes Potential.

Kobehandlung von MMC mit BVDU reduziert die Überexpression von UBE2N und APEX um etwa ein Drittel. Modifikationen von UBE2N beeinflussen die Resistenz gegenüber DNA-Schäden. APEX-Nuklease ist ein DNA-Reparatur Enzym. Blockade der APEX-Expression verdoppelte die Zelllyse und erhöhte DNA-Brüche.

Zu 3) BVDU induziert DT-Diaphorase (Referenz Beispiel 3). Diese besitzt zwei für die Chemotherapie wichtige Eigenschaften. Sie aktiviert zum einen Zytostatika aus der Klasse der Chinone und reduziert zum anderen unspezifische toxische Effekte, die auf der Entstehung reaktiver Sauerstoffspezies beruhen.

Der Ausfall des DT-D Gens führt durch reduzierte p53 und p73 Expression zu myeloischer Hyperplasie und dementsprechend zu reduzierten Apoptose-Raten. Dies stimmt mit der Beobachtung überein, dass ein multifaktorieller "multidrug resistance" Phänotyp von Tumorzellen einen Abfall und keinen Anstieg der DT-Diaphorase Expression beinhaltet. Interessanterweise stabilisiert die DT-D Enzym Aktivität auch die Lymphozyten-Populationen. Dieser Effekt könnte sich günstig auf die Stabilisierung des Immunsystems der Patienten während der Chemotherapie auswirken.

Viele Zytostatika wie z.B. DOX und MXA stören den Redox Status und die mitochondriale Atmung der Krebszelle. Dies führt zur Erzeugung reaktiver Sauerstoff Spezies (ROS). Betroffen von der plötzlichen Anhäufung an ROS ist nicht nur die Krebszelle, sondern auch alle anderen Zellen, wodurch unerwünschte Nebenwirkungen bei der Chemotherapie auftreten.

DT-D inaktiviert ROS und schützt so Zellen vor unspezifischen ROS- und elektrophilen Attacken. Als Indiz für diese Wirkung von BVDU auf die Minderung unerwünschter Nebenwirkungen bei der Chemotherapie seien in Beispiel 4 die Gewichtszunahme von Doxorubicin + BVDU-behandelten Ratten aufgeführt. DOX Alleinbehandlung führt wegen der toxischen Nebenwirkungen zu Gewichtsabnahmen. Sicher ist, dass durch BVDU nur die Nebenwirkungen (möglicherweise die für DOX charakteristische Kardiotoxizität) vermindert werden, nicht jedoch die toxischen Wirkungen auf den Tumor.

Zu 4) Durch veränderte Expression verschiedener Enzyme in der Erholungsphase wird die zytostatische Wirkung auch in Abwesenheit eines Zytostatikums aufrechterhalten. Wie in Beispiel 5 zu sehen ist, wird die Expression von acht Genen erhöht, die von sechs Genen erniedrigt.

Die Genprodukte beeinflussen die Formation von Mikrofilamenten, die Differenzierung, die Signal Transduktion und die ATP Generierung.

Der erfindungsgemäße Gegenstand wird anhand der folgenden Figuren und Beispiele näher erläutert.
- Figur 1: zeigt die Wirkung eines Zytostatikums alleine und in Kombination mit BVDU auf die Anzahl der AH13r-Zellen.
- Figur 2: zeigt im Vergleich zu Figur 1 die Ergebnisse mit Doxorubicin (DOX), Mitoxantron (MXA) und Methotrexat (MTX).
- Figur 3: zeigt eine Western-Blot-Analyse zur Untersuchung der "survival pathways" mit Doxorubicin (DOX).
- Figur 4: zeigt zu Figur 3 entsprechende Untersuchungen mit Mitomycin (MMC).
- Figur 5: zeigt die Ergebnisse der Messung der DT-Diaphorase (DT-D), wobei Doxorubicin (DOX) alleine bzw. zusammen mit BVDU eingesetzt wurde.
- Figur 6: zeigt zu Figur 5 entsprechende Untersuchungen mit Methotrexat (MTX).

### Beispiel 1:

BVDU-Behandlung erhöht die Empfindlichkeit von AH13r Sarkomzellen gegenüber Chemotherapie-induzierter Apoptose. Diese Wirkung bleibt auch nach Absetzen des Zytostatikums in der s.g. Erholungsphase (recovery) bestehen.

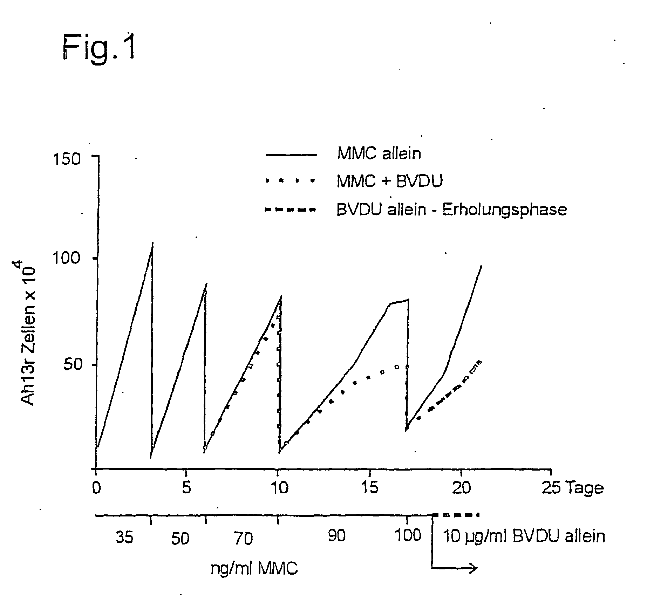

AH13r-Zellen wurden steigende Dosen des Zytostatikums Mitomycin C (MMC) ausgesetzt. BVDU, für sich allein gegeben, zeigte keine toxische Wirkung. MMC+BVDU-Behandlung führte nach drei Behandlungszyklen zur Verringerung der Zellzahl im Vergleich zur Behandlung mit MMC allein. Diese Hemmwirkung blieb auch nach Absetzen des Zytostatikums im nächsten Zyklus, in der s.g. Erholungsphase (recovery) bestehen. Die Zellen ohne MMC und BVDU wuchsen ungehemmt weiter. Diejenigen jedoch, die weiterhin BVDU erhielten, wurden in ihrem Wachstum stark gehemmt (s. Fig. 1).

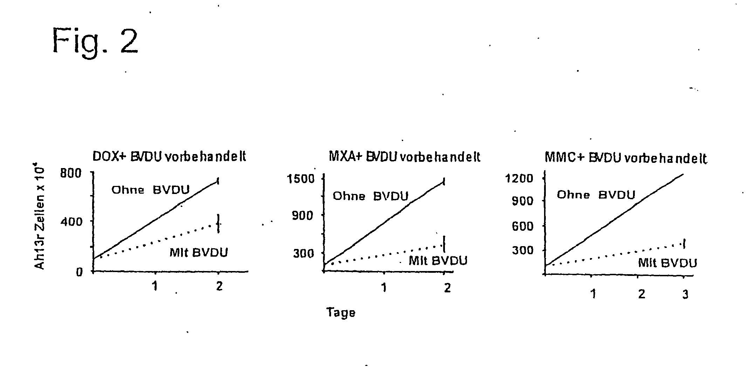

Entsprechende Resultate wurden mit Methotrexat (MTX), Doxorubicin (DOX) und Mitoxantron (MXA) erzielt (s. Fig. 2).

Der Nachweis, dass die Verringerung der Zellzahl auf Apoptose beruht, wurde mittels Hoechst 33258 / Propidiumiodid (Hopi) Doppelfärbung nachgewiesen.

### Referenz:Beispiel 2:

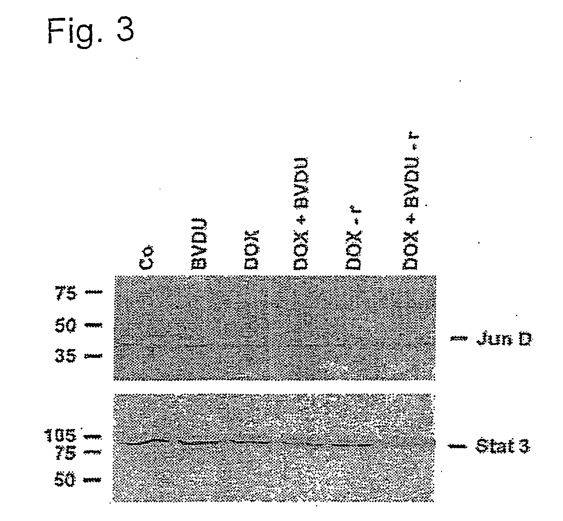

Wir untersuchten verschiedene "survival pathways" mittels Western Blot Analyse. Die Analysen wurden nach Standardmethoden durchgeführt, wie sie in Sambrook et al., 2001, Molecular Cloning (3rd ed.) beschrieben wird. Antikörper Verdünnungen: P-STAT3 (Cell Signaling) 1:500, JUN-D (Santa Cruz, California) 1:1,000. Die obere der beiden JUN-D-Banden zeigt die "full length isoform" und die untere Bande die "truncated isoform", die 48 Aminosäuren kürzer ist. Beide Isoformen können die Transkription aktivieren, die "full length"-Variante ist jedoch effektiver als die "truncated"-Isoform (vgl. Fig. 3).

Der densitometrisch ermittelte Gehalt an Onkogen-Proteinen JUN-D and STAT3 war nach DOX-Behandlung in der Erholungsphase (r = recovery phase) um ein Viertel bzw. zwei Drittel reduziert. In der "recovery" wird nur BVDU gegeben, kein Zytostatikum.

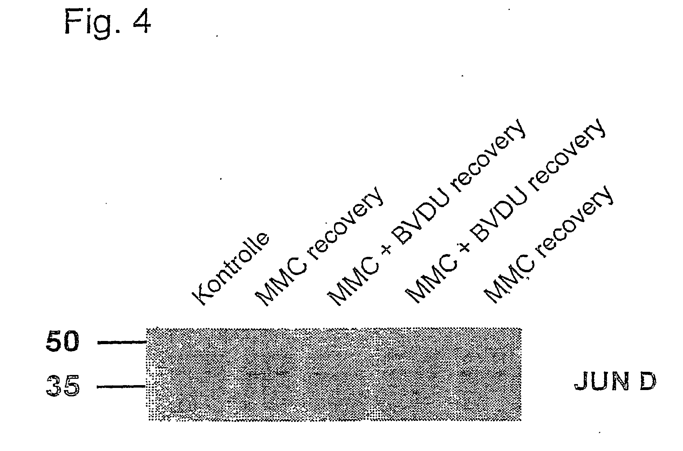

Ein entsprechendes Ergebnis wurde in den Untersuchungen mit Mitomycin C (MMC) erzielt (s. Fig. 4).

Im Versuch mit Mitomycin C (MMC) bewirkte BVDU, in der "recovery" gegeben, eine völlige Hemmung der MMC induzierten JUN-D-Überexpression auf das Kontrollniveau.

### Beispiel 3:

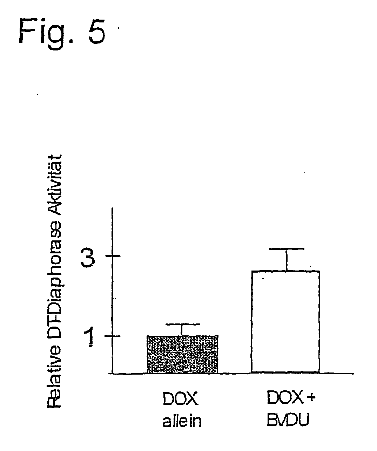

Die Messung der DT-diaphorase (DT-D) erfolgte als Dicoumarol-inhibierbare NAD(P)H: Dichlorphenolindophenol Reduktase, wie in Hodnick et al., Anal. Biochem 252(1), 1997, 165-168 beschrieben. Wir untersuchten Extrakte einer gleichen Anzahl von Zellen, die mit DOX +/- BVDU behandelt worden waren, auf DT-D-Aktivität. Mit BVDU behandelte Zellen zeigten eine annähernd dreifache DT-D-Aktivität als die Zellen aus der Kontrollgruppe oder der Gruppe der alleine mit DOX behandelten Zellen (s. Fig. 5). (Referenz)

Entsprechende Ergebnisse wurden mit Mitoxantron (MXA) und Methotrexat (MTX) erzielt. BVDU allein erhöht die DT-D Aktivität konstant, teilweise aber nur schwach.

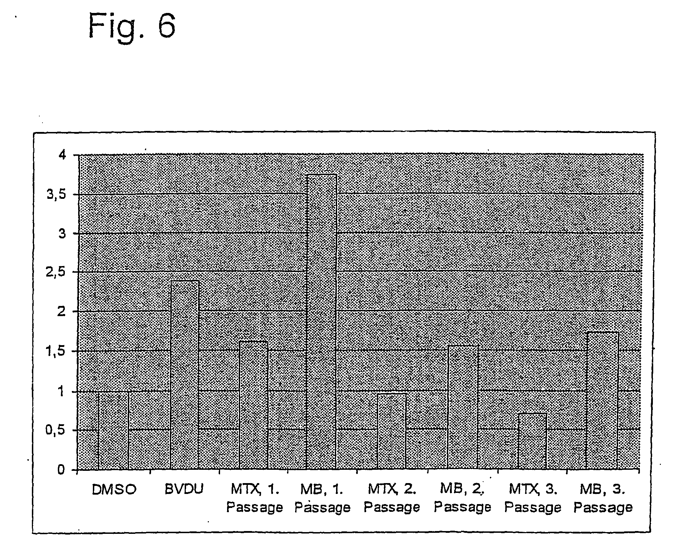

Die Ergebnisse mit Methotrexat (MTX) und menschlichen K562 Tumorzellen sind in Fig. 6 aufgeführt. MB bedeutet MTX + BVDU. Passage bedeutet Verdünnung und Umsetzung der Zellen zum weiteren Wachstum. Auf der Y-Achse ist die relative DT-D Aktivität aufgezeichnet.

### Beispiel 4:

Die Verringerung toxischer Nebenwirkungen von Doxorubicin (DOX) konnte im Versuch mit Ratten gezeigt werden (s. Tabelle 1). SD-Ratten wurden mit Dimethybenzanthrazen (DMBA) behandelt. Die hierdurch induzierten Tumoren wurden durch DOX-Behandlung (1 mg/kg) in ihrem Wachstum gehemmt. Während der Behandlung und einen Tag nach jeder Behandlung, also in der Erholungsphase (recovery phase) erhielten die Tiere jeweils 15 mg/kg BVDU.

**Tabelle 1**

| Durchschnitt der Daten von 5-8 Ratten | Relative Tumorgröße | relatives Tiergewicht | Relative Tumorgröße | relatives Tiergewicht |
|---|---|---|---|---|
| | Tag 1 | Tag 1 | Tag 16 | Tag 16 |
| Kontrolle | 1 | 0 | 6 | + 7 % |
| DOX allein (Referenz) | 1 | 0 | 1.5 | - 7 % |
| DOX + BVDU | 1 | 0 | 1 | + 7 % |

### Beispiel 5:

Auflistung der durch die Behandlung mit Basenanaloga und Mitomycin C beeinflussten Proteine. Die Ergebnisse der Durchführung einer zweidimensionalen Gelelektrophorese sind in der folgenden Tabelle 2 zusammengefasst.

**Tabelle 2**

| **Protein** | **DMSO Kontrolle** | **BVDU allein** |
|---|---|---|
| DEAD/H BOX 1; DDX1 | 0.88 | 0.332 |

| | **MMC allein** | **MMC + BVDU** |
|---|---|---|
| MALAT-DEHYDROGENASE, LÖSLICH; MDH1 | 0.418 | 1.359 |
| MYOSIN, HEAVY CHAIN 1, NORMALE ÄHNLICHKEIT, ERWACHSENER; MYH1 | 0.182 | 0.588 |
| UBIQUITIN-KONJUGIERENDES ENZYM E2N; UBE2N | 0.669 | 0.178 |
| APURINIC ENDONUCLEASE; APE; APE1; APEX | 0.363 | 0.14 |

| | **MMC "recovery", Weiterkultivierung ohne MMC und BVDU** | **MMC + BVDU "recovery" Weiterbehandlung durch BVDU allein** |
|---|---|---|
| PLATELET-AKTIVIERENDES FAKTOR ACETYL-HYDROLASE, ISOFORM 1B, ALPHA UNTEREINHEIT; PAFAH1B1 | 0.219 | 0.619 |
| U5 snRNP-SPECIFIC PROTEIN, 116-KD | 0.2 | 0.523 |
| HÄMOGLOBIN-BETA LOCUS; HBB | 0.088 | 0.502 |
| HÄMOGLOBIN-ALPHA LOCUS 1; HBA1 | 0.054 | 0.316 |
| ACTIN, BETA; ACTB | 0.163 | 0.451 |
| Ähnlich zu BETA-ACTIN | 0.096 | 0.357 |
| ACTIN ähnlich | 0.112 | 0.398 |
| TROPOMODULIN 2; TMOD2 | 0.095 | 0.28 |
| SUCCINAT-DEHYDROGENASE-KOMPLEX, UNTEREINHEIT A, FLAVOPROTEIN; SDHA | 0.255 | abwesend |
| PYRUVAT-DEHYDROGENASE-KOMPLEX, E1-ALPHA POLYPEPTID 1; PDHA1 | 1.751 | 0.533 |
| TUBULIN, BETA-5 | 4.705 | 1.553 |
| POLY(rC)-BINDENDES PROTEIN 2; PCBP2 | 0.912 | 0.234 |
| MALAT-ENZYM 2; ME2 | 0.972 | 0.322 |
| Mini-Chromosom Erhaltung unzureichend 7; MITOTIN, ZELLKLASSE ZYKLUS-ÄHNLICH 1; CDCL1 | 0.374 | 0.119 |

## Patentansprüche

1. Verwendung von (E)-5-(2-Bromovinyl)-2'-deoxyuridin (BVDU), und/oder dessen Salze zur Herstellung eines Arzneimittels zur Verstärkung der apoptotischen Wirkung von Zytostatika nach Abschluss der Chemotherapie bei der mindestens ein Zytostatikum in Verbindung mit BVDU, und/oder dessen Salzen eingesetzt worden ist, ohne die zusätzliche Gabe eines Zytostatikas.

2. Verwendung nach Anspruch 1,
**dadurch gekennzeichnet, dass** bei der Chemotherapie über einen definierten Zeitraum steigende Dosen des Zytostatikums in Kombination mit BVDU, und/oder dessen Salzen allein eingesetzt worden ist und im Anschluss an die Chemotherapie, d.h. in einer Erholungsphase, BVDU, und/oder dessen Salze allein eingesetzt wird.

3. Verwendung nach Anspruch 2,
**dadurch gekennzeichnet, dass** die Dauer der Erholungsphase von 3 bis zu 10 Tagen beträgt.

4. Verwendung nach einem der Ansprüche 2 oder 3,
**dadurch gekennzeichnet, dass** die Dauer des Chemotherapiezyklus von 8 bis zu 30 Tagen beträgt.

5. Verwendung nach Anspruch 1,
**dadurch gekennzeichnet, dass** als BVDU ein Prodrug der allgemeinen Formel I 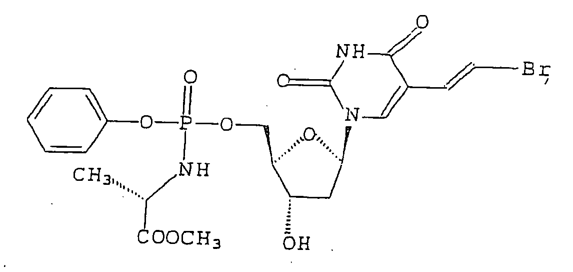 eingesetzt wird.

6. Verwendung nach mindestens einem der Ansprüche 1 bis 5,
**dadurch gekennzeichnet, dass** BVDU, und/oder dessen Salze in einer Dosierung eingesetzt werden, die eine Blutkonzentration zwischen 0,02 und 50 µg/mL ermöglicht.

## Claims

1. Use of (E)-5-(2-bromovinyl)-2'-deoxyuridin (BVDU) and/or its salts for producing a medicine for reinforcing the apoptotic effect of cytostatica following chemotherapy, whereby at least one cytostaticum in connection with BVDU and/or its salts is used without the additional administration of a cytostatica.

2. Use according to Claim 1, **characterised in that** increasing doses of the cytostaticum in combination with BVDU and/or its salts is used on its own during chemotherapy over a defined period of time, and **in that** BVDU and/or its salts is used on its own following the chemotherapy, e.g. during a recovery phase.

3. Use according to Claim 2, **characterised in that** the duration of the recovery phase is between 3 and 10 days.

4. Use according to one of the Claims 2 or 3, **characterised in that** the duration of the chemotherapy cycle is between 8 and 30 days.

5. Use according to Claim 1, **characterised in that** a pro-drug with the general formula I 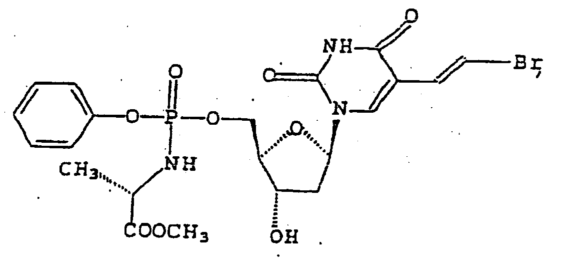 is used as BVDU.

6. Use according to at least one of the Claims 1 to 5, **characterised in that** the BVDU and/or its salts are used in a dose that enables a blood concentration of between 0.02 and 50 µg/mL.

## Revendications

1. Utilisation de la (E)-5-(2-bromovinyl)-2'-désoxyuridine (BVDU) et/ou de ses sels pour la fabrication d'un produit pharmaceutique pour renforcer l'effet apoptotique de cytostatiques à la fin de la chimiothérapie, dans laquelle on utilise au moins un cytostatique conjointement avec la BVDU et/ou ses sels, sans l'administration supplémentaire d'un cytostatique.

2. Utilisation selon la revendication 1, **caractérisée en ce que** l'on utilise en chimiothérapie sur une période de temps définie des doses croissantes, seules, du cytostatique en combinaison avec la BVDU et/ou ses sels, et **en ce que** l'on utilise à la fin de la chimiothérapie, c'est-à-dire lors d'une phase de repos, la BVDU, et/ou ses sels, seule.

3. Utilisation selon la revendication 2, **caractérisée en ce que** la durée de la phase de repos est de 3 à 10 jours.

4. Utilisation selon l'une quelconque des revendications 2 ou 3, **caractérisée en ce que** la durée du cycle de chimiothérapie est de 8 à 30 jours.

5. Utilisation selon la revendication 1, **caractérisée en ce que** l'on utilise, comme BVDU, un promédicament de formule générale I :

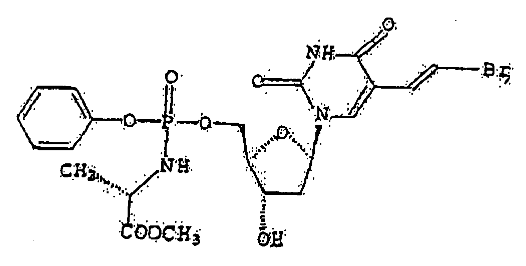

6. Utilisation selon au moins l'une quelconque des revendications 1 à 5, **caractérisée en ce que** l'on utilise la BVDU et/ou ses sels selon un dosage qui permette une concentration sanguine comprise entre 0,02 et 50 µg/ml.
